Europäisches Patentamt

European Patent Office

Office européen des brevets

(19)

(11) Publication number: **0 138 075**
A1

(12) # EUROPEAN PATENT APPLICATION

(21) Application number: **84111001.8**

(22) Date of filing: **14.09.84**

(51) Int. Cl.⁴: **C 12 N 15/00,** C 12 N 9/00, C 12 N 1/20 // (C12R1/07, 1:185)

(30) Priority: **16.09.83 US 532765**

(43) Date of publication of application: **24.04.85** Bulletin 85/17

(84) Designated Contracting States: **AT BE CH DE FR GB IT LI LU NL SE**

(71) Applicant: **SYNERGEN ASSOCIATES, INC., 1885 33rd Street, Boulder Colorado 80301 (US)**

(72) Inventor: **Liao, Hans H., 6530 Barnacle Street, Boulder, CO 80301 (US)**
Inventor: **McDonell, Michael W., 4262 Redwood Place, Boulder, CO 80301 (US)**
Inventor: **Gold, Lawrence M., 955 8th Street, Boulder, CO 80301 (US)**
Inventor: **Hirsh, David I., 270 Green Rock Drive, Boulder, CO 80301 (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath, Maximilianstrasse 58, D-8000 München 22 (DE)**

(54) A method for the generation and detection of enzyme variants with enhanced themostability and activity.

(57) A method for transforming thermophilic microorganisms. A method for transforming thermophilic microorganisms is disclosed comprising: (a) forming a protoplast from a thermophilic microorganism; (b) introducing a plasmid chimera into the protoplast; and (c) causing regeneration of the cell wall of the protoplast. A novel regeneration medium is employed in the transformation method. This method involves the use of novel plasmid chimera. The plasmid chimeras include a newly discovered thermostable promoter. A method of isolating promoters capable of functioning at elevated temperatures is included. A method for selecting thermostable variants of gene products of cloned genes in thermophilic microorganisms is also disclosed.

EcoRI           Hind III

AA TT C  AA GC TT

   G TT CG  AA TT AA

EP 0 138 075 A1

ACTORUM AG

## A METHOD FOR THE GENERATION AND DETECTION OF ENZYME VARIANTS WITH ENHANCED THERMOSTABILITY AND ACTIVITY

The present invention relates generally to the transformation of thermophilic microorganisms, plasmid chimeras and their production, and a process for producing certain useful biological products using the same. Additionally, the present invention relates to a method for creating thermostable variants of useful enzymes. More specifically, the invention relates to plasmid vectors which provide the thermostable origins of replication and promoters necessary to express mesophilic, cryophilic, thermophilic or synthetic genes at elevated temperatures, a method for isolating thermostable promoters, methods of so expressing mesophilic, cryophilic, thermophilic or synthetic genes at elevated temperatures, and a method for screening transformed microorganisms to select those microorganisms which express desired phenotypes.

Microorganisms which are capable of growth at elevated temperatures, hereinafter referred to as "thermophilic microorganisms" or "thermophiles", are of industrial importance. Of special industrial importance are the nonpathogenic, thermophilic microorganisms that are facultative anaerobes, capable of growth at temperatures of up to about 70°C. These microorganisms may be grown in large-scale fermentation vats that require less expenditure of energy for cooling and aeration than do comparable fermentation processes using microorganisms capable only of growth at lower temperatures or requiring high dissolved oxygen concentrations. In addition, the danger of contamination of the fermentation vat by undesired foreign organisms is substantially reduced when thermophilic microorganisms are used due to the elevated growth temperatures.

Bacillus stearothermophilus is one such facultatively anaerobic, thermophilic microorganism. In addition, this microorganism actively secretes proteins such as proteases, which themselves are commercially useful. The development of plasmid transformation and cloning systems in various Bacillus species has expanded the role of these organisms in industrial settings. In particular, it appears possible to take advantage of the ability of these bacteria to secrete proteins to establish a simplified process for obtaining specific products of cloned genes which do not require the purification steps required for recovery of non-secreted products.

In the past, methods of transformation and plasmid vectors and chimeras suitable for such transformation in mesophilic microorganisms of the genus Bacillus have been identified. An example of one such method is shown in "High Frequency Transformation of Bacillus subtilis Protoplasts by Plasmid DNA"; S. Chang and S. Cohen, Mol. Gen. Genet. 168, 111-115 (1979). However, the ability to transform mesophilic bacteria does not provide the industrially desirable opportunities that would exist if the ability to transform thermophilic micro-organisms existed.

Transformation of <u>Bacillus stearothermophilus</u> is disclosed in "Transformation of <u>Bacillus stearothermophilus</u> with Plasmid DNA and Characterization of Shuttle Vector Plasmids between <u>Bacillus stearothermophilus</u> and <u>Bacillus subtilis</u>", Imanaka, <u>et al</u>., Journal of Bacteriology, March 1982, pp. 834-830. Imanaka's process involves embedding the newly transformed microorganisms in a regeneration agar to allow regeneration of the cell wall. Using this process, transformant frequency may be scored in five days. Using the process of the present invention, which does not embed the newly transformed microorganisms in regeneration agar and uses a novel transformation medium, transformation frequency can be scored after two days.

Schuichi and Imanaka also disclose the transformation of thermophilic microorganisms in European Patent Application 82301150.7. The present invention sets forth a method for transformation of thermophilic microorganisms which uses novel plasmid chimeras, uses novel regeneration media, and obtains transformants capable of being scored in two days.

Novel plasmids have been discovered which provide cloning vectors for the insertion of genetic material into thermophilic microorganisms. The novel plasmids include thermostable origins of replication and thermostable promoters. The present invention also includes a method of transforming thermophilic microorganisms by the insertion of the novel plasmids or known mesophilic plasmids into the organisms, which results in the transformants expressing the acquired phenotype in a shortened time period through the use of a newly discovered regeneration medium. Further, the present invention provides a process for the isolation of thermostable variants of formerly cryophilic, mesophilic, thermophilic or synthetic genetic material capable of being expressed and producing gene products that are functional at elevated temperatures.

Specifically, there have been discovered thermostable plasmids capable of replication and phenotypical expression of genetic material obtained from non-thermophilic organisms. In addition, thermostable plasmids capable of replication and phenotypical expression of synthetic genetic material have been discovered as well as plasmids capable of expression of thermophilic genetic material at a higher temperature.

The plasmid pBST1 has been discovered, as have the plasmid chimeras pBST2, pBST-6, pBST2-6TK, pSHW9, pBST8, pCV1, pCV3, pBST110 and pRMS10. Further, a promoter for initiating expression of non-thermophilic genes at elevated temperatures has been discovered. A method for isolation of a promoter capable of operating at elevated temperatures has been discovered comprising: (a) construction of a plasmid chimera useful for determining whether an inserted piece of DNA is a promoter capable of functioning at an elevated temperature, the plasmid chimera comprising: (1) a genetic sequence capable of expressing a detectable phenotype; (2) a ribosome binding site capable of operation at elevated temperatures; and (3) an insertion site wherein a piece of DNA may be inserted such that the piece of DNA would, if capable, act as a promoter for the genetic sequence; (b) inserting the piece of DNA suspected of being a promoter capable of operating at elevated temperatures into the insertion site; (c) transforming the plasmid chimera into a thermophilic microorganism; and (d) identifying the microorganisms which express the phenotype corresponding to the genetic sequence.

Also, there has been discovered a method for the transformation of thermophilic microorganisms comprising the steps of: (a) forming a protoplast from a thermophilic microorganism; (b) introducing a plasmid chimera which contains genetic material from a source other than a thermophilic organism into the protoplast; and (c) causing regeneration of the cell wall of the protoplast.

Further, there has been discovered a method of producing a stable biological product at elevated temperatures corresponding to the product of non-thermophilic genetic material comprising: (a) transforming a thermophilic microorganism by the method comprising: (1) forming a protoplast from a thermophilic microorganism; (2) introducing into a protoplast a plasmid chimera which contains genetic material from a source other than a thermophilic organism; and (3) causing regeneration of the cell wall of the protoplast; and (b) placing the transformed thermophilic microorganism on a growth medium sufficient to cause the microorganism to multiply and to cause expression at elevated temperatures of a stable biological product corresponding to the introduced genetic material.

Finally, a method of creating thermostable variants of biological products has been discovered, comprising: (a) inserting into a plasmid chimera a DNA sequence encoded for production of the biological product; (b) causing the DNA sequence to be mutagenized; (c) transforming a microorganism capable of growth at an elevated temperature with the plasmid chimera; and (d) growing the transformed microorganism at elevated temperatures to identify microorganisms which express thermostable variants of the biological product.

Additional objects and advantages of the invention will be set in part in a description which follows, and in part will be obvious from the description, or may be learned by practice of the invention. The objects and advantages may be realized and attained by means of the instrumentalities and methods particularly pointed out in the appended claims.

It is understood that both the foregoing general description and the following detailed description are exemplary and explanatory only and are not restrictive of the invention as claimed.

The accompanying drawings, which are incorporated in and constitute a part of this specification, illustrate preferred embodiments of the invention and, together with the description, serve to explain the principles of the invention.

FIGURE 1 is a legend defining sources of DNA segments illustrated in Figures 2-9.

FIGURE 2 is a DNA base pair sequence representative of the synthetic oligonucleotide linker of the preferred embodiment of the invention.

FIGURE 3 is a restriction map of plasmid chimera pBST2.

FIGURE 4 is a restriction map of plasmid chimera pBST2-6.

FIGURE 5 is a restriction map of plasmid chimera pSHW9.

FIGURE 6 is a restriction map of plasmid chimera pBST8.

FIGURE 7 is a restriction map of plasmid chimera pCV1.

FIGURE 8 is a restriction map of plasmid chimera pCV3.

FIGURE 9 is a restriction map of plasmid chimera pBST110.

References will now be made in detail to the presently preferred embodiments of the invention, which together with the following examples, serve to explain the principles of the invention.

As noted above, the process of the present invention provides novel plasmids to transform thermophilic microorganisms to obtain expression of formerly cryophilic, mesophilic or thermophilic phenotypes at an elevated temperature. The present invention also employs novel plasmids to transform thermophilic microorganisms to obtain expression of synthetic genetic material at an elevated temperature. Additionally, the present invention provides promoters that function at elevated temperatures and a process for the isolation of the same. The present invention further employs a novel method of transforming thermophilic microorganisms which results, _inter alia_, in the transformants expressing the acquired phenotype in a shortened time period through the use of newly discovered regeneration medium. Further, the process of the present invention provides a method for creating thermostable variants of useful biological products.

The following terms, used throughout this application, are intended to have the following meanings:

Plasmid chimera - a recombinant plasmid molecule containing genes capable of expressing at least one phenotypical property;

Plasmid vector or cloning vector - a plasmid molecule into which DNA segments may be inserted to allow the transfer of these segments into microorganisms in which the plasmid molecule is able to be propagated.

Thermophile or thermophilic - capable of growing or functioning at a temperature above about 50°C;

Mesophile or mesophilic - capable of growing or functioning at temperatures ranging from about 15°C to below about 50°C;

Cryophile or cryophilic - capable of growing or functioning at temperatures of about 15°C or less.

Synthetic genetic material - a DNA sequence, constructed outside of a living organism, which contains base pairs in a sequence appropriate to direct a cell to produce a desired biological product.

Synthetic oligonucleotide linker - a DNA sequence, constructed outside a living organism, which provides cloning sites (restriction endonuclease recognition sites).

Elevated temperature - a temperature of about 50°C or higher.

$Kan^R$ - a DNA sequence which contains base pairs situated in an order appropriate to direct a cell to express, as a portion of its phenotype, resistance to the antibiotic kanamycin.

Amp$^R$ - a DNA sequence which contains base pairs situated in an order appropriate to direct a cell to express, as a portion of its phenotype, resistance to the antibiotic ampicillin.

Cam$^R$ - a DNA sequence which contains base pairs situated in an order appropriate to direct a cell to express, as a portion of its phenotype, resistance to the antibiotic chloramphenicol.

CAT - the enzyme chloramphenicol acetyl transferase.

CAT gene - the DNA sequence which contains the base pairs situated in an order that, if expressed, produces CAT.

Thermostable Plasmid Chimeras

The present invention involves transformation of thermophilic organisms with novel plasmid chimeras capable of introducing non-thermophilic genetic material into those organisms and expression of the attendant gene products at elevated temperature. Thus, the present invention includes a thermostable plasmid capable of replication and phenotypical expression in a thermophilic organism of genetic material obtained from a non-thermophilic organism, comprising: (a) a thermostable origin of replication; (b) genetic material obtained from a non-thermophilic organism; and (c) a thermostable promoter located in a position to direct transcription of such genetic material. The present invention also includes a thermostable plasmid capable of replication and phenotypical expression of synthetic genetic material. In addition, the present invention includes a thermostable plasmid which additionally comprises a synthetic oligonucleotide linker which may be inserted into the plasmid to create unique ligation sites suitable for the addition into the plasmid of additional genetic material.

Thermostable Origin of Replication

The preferred thermostable origin of replication of the present invention is isolated from a large, cryptic plasmid found in <u>Bacillus stearothermophilus</u> strain NRRL 1102, growing at 70°C. This strain was obtained from Northern Regional Research Center, U.S. Department of Agriculture, Peoria, Illinois. The plasmid contains approximately 80 kilo bases (kb). A thermostable origin of replication has been discovered on a fragment of 1.4 kb obtained by total <u>Cla</u>I digestion of this large cryptic plasmid. This origin of replication was also isolated on a fragment of 3.6 kb obtained by RI digestion of the cryptic plasmid.

<u>Isolation of a Thermostable Promoter That Functions at Thermophilic Temperatures</u>

The ability to isolate a promoter capable of functioning at elevated temperatures which will direct expression, at elevated temperatures, of desired phenotypes is critical to successful industrial use of the method of transformation of thermophilic microorganisms disclosed herein. The present invention includes, therefore, a method for isolation of such a promoter comprising: (a) construction of a plasmid chimera useful for determining whether an inserted piece of DNA is a promoter capable of functioning at elevated temperatures, the plasmid chimera comprising: (1) a genetic sequence capable of expressing a detectable phenotype; (2) a ribosome-binding site capable of operation at thermophilic temperatures; and (3) an insertion site wherein a piece of DNA may be inserted such that the DNA would, if capable, act as a promoter for said genetic sequence; (b) inserting the pieces of DNA suspected of being promoter capable of functioning at an elevated temperatures into the insertion site; (c) transforming the plasmid chimera into a thermophilic microorganism; and (d) selecting those microorganisms which express, at an elevated temperature, the phenotype corresponding to the genetic sequence.

In the method of the present invention, it is preferred that the plasmid chimera used is pBST110, described more fully below. Plasmid chimera pBST110 has an origin of replication, ribosome binding site, and CAT gene all capable of functioning at elevated temperatures. The piece of DNA suspected of being a promoter is inserted in the opening created by digestion of pBST110 by the restriction enzymes NarI or BclI. The preferred method of selecting DNA pieces suspected of containing promoters that function at elevated temperatures is to obtain DNA native to a thermophilic microorganism, such as chromosomal or plasmid DNA growing in a thermophilic environment. The thermophilic microorganism from which DNA is obtained may be of the same species as the microorganism into which the plasmid chimera is to be transformed.

The DNA is removed from the thermophilic microorganisms by any method known to those skilled in the art which does not disrupt the ability of the DNA to function. An acceptable method includes, but is not limited to, the procedure disclosed in "Advanced Bacterial Genetics", by R. W. Davis, D. Botstein and J. R. Roth, Cold Spring Harbor Laboratory, Cold Spring Harbor, New York, specifically incorporated herein by reference.

The DNA is then digested using, for example, MspI, if the resultant fragments are to be inserted into the NarI site of plasmid pBST110 described more fully below, or using Sau3AI, if the resultant fragments are to be inserted into the BclI site of pBST110, and shotgun cloned into the plasmid chimera.

The resultant plasmid chimeras are transformed into the thermophilic microorganism by the method for transformation of thermophilic microorganisms disclosed below. The selection of the microorganisms containing the desired promoter is accomplished by the detection of microorganisms which exhibit the phenotype encoded for in the genetic sequence of the plasmid chimera when observed at an elevated temperature. In a preferred embodiment of the present invention, when the genetic sequence is expressed as the CAT enzyme, the transformed microorganisms are grown, at an elevated temperature, in the presence of chloramphenicol. Microorganisms that survive at the elevated temperature and, in the presence of chloramphenicol, do so by producing CAT. These are microorganisms that have been transformed with a plasmid chimera containing a promoter capable of functioning at an elevated temperature.

The plasmid chimera can then be recovered from the transformed microorganism and re-inserted into any other plasmid chimera if it is so desired.

Thermostable Promoter

The promoter included in the present invention is thermostable and capable of directing, at an elevated temperature, the transcription of the DNA coding for any biological product. Once transcribed, cellular components will cause the expression of the phenotype encoded for on the DNA obtained from a non-thermophilic organism or synthetically obtained. It is also possible to use the present invention to direct transcription of genetic material obtained from a thermophilic organism when the genetic material is included in a plasmid chimera transformed into a microorganism growing at a more elevated temperature. The promoter of the preferred embodiment is obtained by digestion, using Sau3AI restriction enzyme, of chomosomal DNA obtained from Bacillus stearothermophilus, strain 1174 and is included in plasmid pRMS 10 described below in a position to regulate expression of the CAT gene contained in that plasmid.

In the preferred embodiment, the promoter is positioned so as to enable it to direct the transcription of the genetic material in the plasmid chimera into mRNA.

Synthetic Oligonucleotide Linker

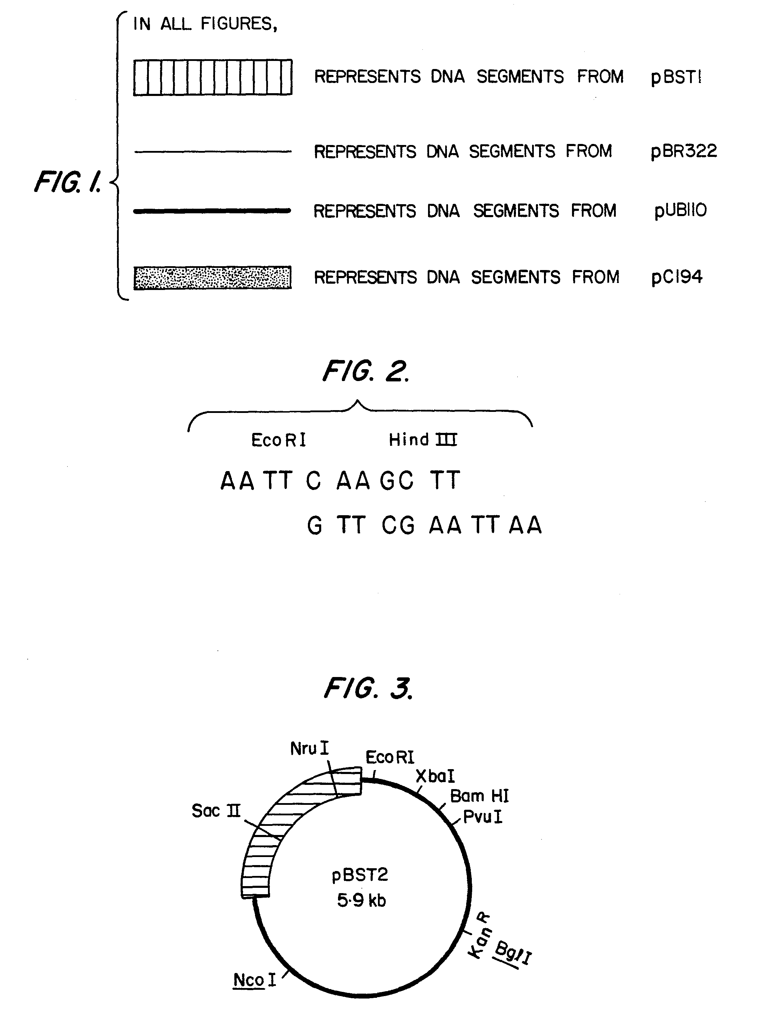

It is preferred to use the synthetic oligonucleotide linker diagrammed in Fig. 2 (using conventional nucleotide abbreviations ) which provides unique sites for restriction endonucleases to attach and cause a cleavage in the plasmid. The synthetic obligonucleotide linker of Fig. 2 may be obtained by sequentially combining the appropriate DNA base pairs to form a strand of DNA suitable for use as a linker. Preferably, the base pairs are attached to each other by the method disclosed in Caruthers, M. H., Beaucage, S. L., Becker, C., Efcavitch, W., Fisher, E. F., Gallupi, G., Goldman, R., deHaseth, P., Martin, F., Matteucci, M., and Stabinsky, Y., in Genetic Engineering, pp 1-17, eds. Setlow, J.K., and Hollaender, A., Plenum Press, New York, 1982, the disclosure of which is specifically incorporated herein by reference.

The synthetic oligonucleotide linker is positioned in the plasmid chimera so as to provide a useful insertion point for foreign DNA that may be added to the plasmid chimera. If the genetic material desired to be expressed is already a part of the plasmid chimera, the linker might be located so as to provide an insertion point for the addition of a promoter that directs expression of the desired genetic material. The linker could also be inserted at a position which would disrupt the function of an undesired gene, promoter, origin of replication, or other DNA sequence native to the plasmid chimera. Once the linker was so inserted, the restriction sites provided by the linker could serve as insertion sites for any DNA sequences subsequently desired in the plasmid chimera.

Preferred Thermostable Vectors

The following plasmid chimeras are particularly useful in accordance with the present invention. These plasmid chimeras are described herein below and are obtainable as indicated:

(a) pBST1

Plasmid pBST1 is a large, cryptic plasmid isolated from strains of Bacillus stearotheromophilus growing at 70°C. Particularly, pBST1 may be isolated from strain NRRL 1102 obtained from Northern Regional Research Center, U.S. Department of Agriculture, Peoria, Illinois, by techniques known to the art as above described. Plasmid pBST1 is approximately 80 kb in size, as determined by restriction endonuclease analysis and is present at one copy per cell. Plasmid pBST1 may be digested with ClaI into approximately 20 fragments.

(b) pBST2

Plasmid chimera pBST2, as illustrated in Fig. 3, is a plasmid chimera containing a thermostable origin of replication derived from pBST1 and a $kan^R$ marker derived from pUB110 (pUB110, a Bacillus subtilis plasmid, is obtainable from the Bacillus Genetic Stock Center, Columbus, Ohio). To construct pBST2, pUB110 is digested with TaqI. Digestion with TaqI inactivates the pUB110 origin of replication but leaves intact the $kan^R$ gene. Plasmid pBST1 is digested into total ClaI fragments. The pUB110 fragments are ligated with the T4 DNA ligase to the total ClaI digestion fragments of pBST1 to form pBST2.

When tested in Bacillus stearothermophilus, pBST2 yields a thermally-stable transformant conferring kanamycin resistance at 47°C, even after being grown for a period of time at 70°C. However, PBST2 does not exhibit kanamycin resistance at 70°C, due to the inherent thermal instability of the kanamycin nucleotidyltransferase protein. When analyzed by restriction endonuclease digestion and Southern hybridiyation, pBST2 is found to contain a 1.4 kb DNA fragment originating from pBST1 as well as the three TaqI fragments corresponding to intact pUB110 as diagrammed in Fig. 3. It is found, however, that the NcoI site of pUB110 is displaced by approximately 450-bp, an indication that the two minor TaqI fragments of pUB110 are rearranged in pBST2. It has been found that pBST2 is present at one to three copies per cell after having been transformed into Bacillus stearothermophilus.

Plasmid chimera pBST2 is useful to transform Bacillus stearothermophilus to confer kanamycin resistance.

As set forth in Table 1 below, plasmid pBST2 demonstrates enhanced thermostability as compared to pUB110 in B. stearothermophilus. The plasmid pBST2 may be isolated by above described techniques known to the art from the B. stearothermophilus culture 39425 deposited in the American Type Culture Collection, Rockville, Maryland (herinafter "ATCC").

Table I

(Percent of cells retaining plasmid after
20 generations of growth at specified
temperature in the absence of Kanamycin).

| Temperature (°C) | pUB110 | pBST2 |
| --- | --- | --- |
| 47 | 99 | 100 |
| 55 | 36 | 95 |
| 59 | 27 | 98 |
| 63 | 26 | 58 |
| 67 | 1 | 46 |

Plasmid pBST2 has been shown to be more stable at high temperatures than natural Bacillus stearothermophilus plasmids described in Inamaka et al. (Ibid.)

(c) pBST2-6

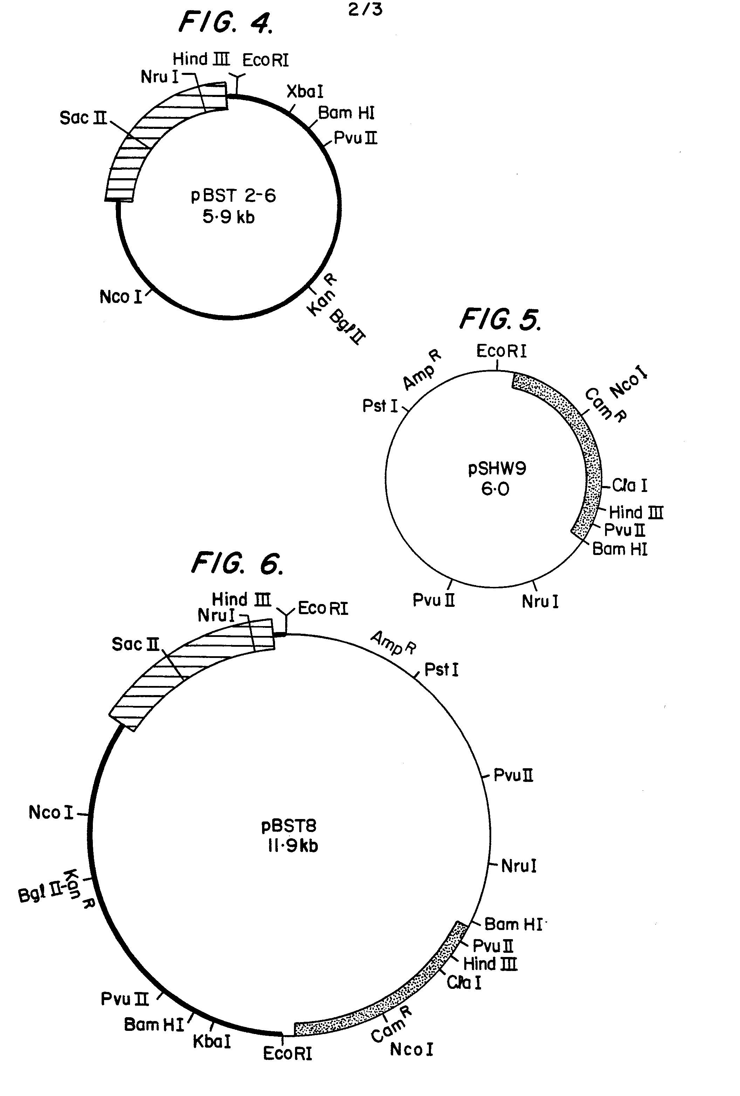

Plasmid chimera pBST2-6, as illustrated in Fig. 4, is prepared by the insertion of the synthetic oligonucleotide linker of Fig. 2 into pBST2 to provide a unique HindIII site in the plasmid chimera adjacent to the EcoRI site. To form pBST2-6, pBST2 is digested with EcoRI. The linker is attached to the pBST2 fragment by ligation with bacteriophage T4 DNA ligase. The addition of this synthetic oligonucleotide linker increases the usefulness of this plasmid chimera as a cloning vector. Plasmid chimera pBST2-6 transforms Bacillus stearothermophilus and confers kanamycin resistance to the transformants. The plasmid pBST2-6 may be isolated by above described techniques known to the art from the B. stearothermophilus culture 39429 deposited in the ATCC.

(d)    pBST2-6TK

Plasmid chimera pBST2-6K is a variant of plasmid chimera pBST2-6 encoding a kanamycin nucleotidyltransferase, the enzymatic activity confering resistance to kanamycin, which is thermostable and active at high temperatures, whereas the corresponding enzyme encoded by pBST2-6 is not stable or active at above about 55°C. This thermostable variant was obtained by selection for a spontaneous mutant that exhibited resistance to kanamycin at 66°C. The plasmid pBST2-6TK may be obtained by above-described techniques known to the art from B. stearothermophilus culture 39426 in the ATCC.

(e)    pSHW9

Plasmid chimera pSHW9, as illustrated in Fig. 5, was created to obtain chloramphenicol resistance by the addition of the gene which codes for the manufacture of chloramphenicol acetyl-transferase (CAT). Plasmid chimera pSHW9 is constructed by combining Escherichia coli plasmid pBR322 (commercially available from Bethesda Research Laboratories, Bethesda, Maryland), and pC194 (obtainable from the Bacillus Genetic Stock Center, Columbus, Ohio), such that the origin of replication of pC194 is destroyed. To construct pSHW9, pC194 is digested with MspI followed by digestion with Sau3AI. The fragments obtained are ligated to fragments obtained by sequential ClaI and BamHI digestions of pBR322. These digestion procedures leave two sets of complementary overlaps; restriction enzymes MspI and ClaI leave complementary overhangs as do Sau3AI and BamHI. Plasmid chimera pSHW9 confers ampicillin and chloramphenicol resistance to E. coli. However, pSHW9 is incapable of replication in organisms of the genus Bacillus. The plasmid pSHW9 may be obtained by above-described techniques known to the art from E. coli culture 39433 in the ATCC:

(f) pBST8

Plasmid chimera pBST8, as illustrated in Fig. 6, is constructed from a combination of pSHW9 and pBST2-6, providing an origin of replication for Bacillus stearothermophilus obtained from pBST2-6 and for E. coli from pSHW9. To form pBST8, pSHW9 and pBST2-6 are each digested with EcoRI and ligated together.

E. coli transformants containing pBST8 acquire simultaneous resistance to ampicillin, kanamycin and chloramphenicol. Plasmid chimera pBST8 transforms both E. coli and Bacillus stearothermophilus and, when isolated from either species, will transform the other one. When present in E. coli, pBST8 will confer resistance to ampicillin, kanamycin and chloramphenicol, but, when present in Bacillus stearothermophilus, the ampicillin resistance derived from pBR322 is not expressed. Plasmid pBST8 may be isolated by above-described techniques known to the art from B. stearothermophilus culture 39427 in the ATCC.

(g) pCV1

To obtain optimal expression of cloned, foreign, mesophilic cryophilic, thermophilic or synthetic genes in thermophilic microorganisms, it is desirable to insert, into the plasmid chimera used, a native promoter. It is preferred, although not necessary, that the native promoter be selected from a member of the thermophilic genus into which the plasmid chimera is ultimately to be introduced. The present invention contemplates a plasmid designed to facilitate identification and isolation of a native promoter suitable for directing expression of cloned, foreign genes in thermophilic microorganisms. This plasmid chimera, pCV1, is useful in the method for isolation of a promoter capable of functioning at an elevated temperature described above.

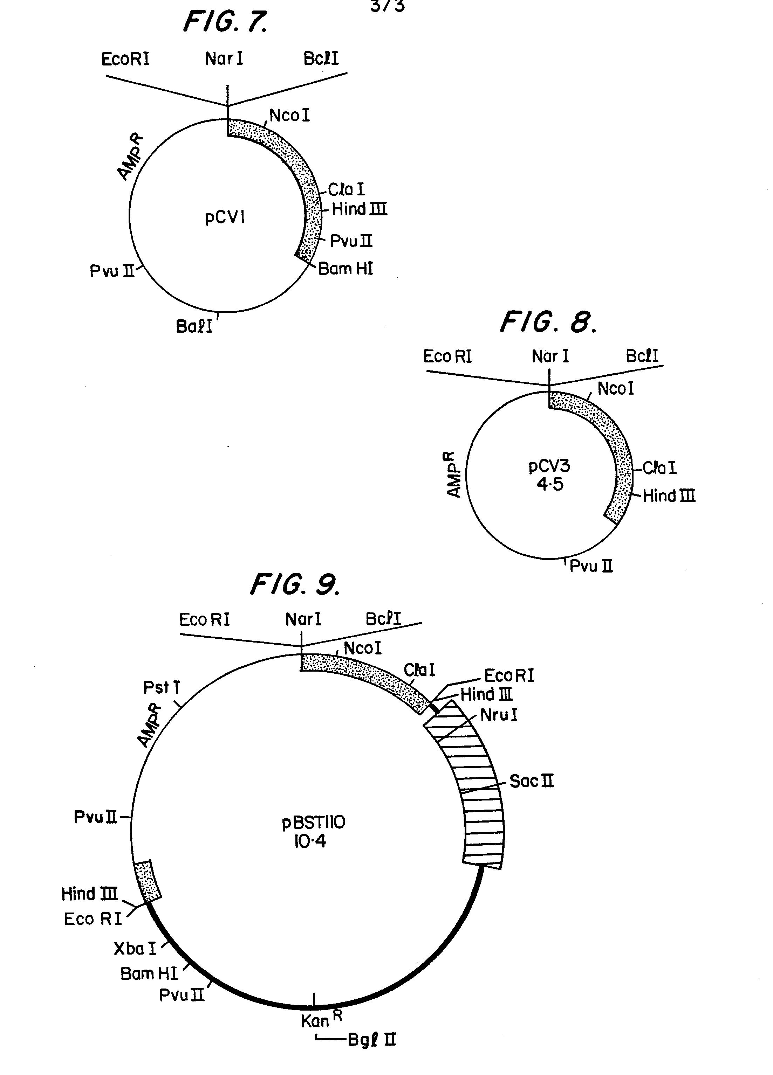

This plasmid chimera is engineered so that a drug-resistance marker is not expressed except when a promoter is inserted at the 5' end of the drug-resistance gene. To construct this plasmid, the CAT gene from pC194 is cloned into pBR322 to generate the plasmid chimera pSHW9. In addition, a 452-bp DNA fragment containing the ribosome-binding site and the amino-terminal portion but not the natural promoter of the same CAT gene are isolated from an MnlI plus NcoI digestion of pC194 by fractionation on an agarose gel. The MnlI end of the 452-bp piece constitutes a blunt end. This blunt end is ligated with a synthetic oligonucleotide linker consisting of the restriction sites for EcoRI, NarI and BclI. The products of this ligation reaction are re-digested with EcoRI, NarI and BclI. The products of this ligation reaction are re-digested with EcoRI and NcoI to break down multimers. The EcoRI-NcoI fragment of the 464-bp segment finally obtained contains the three restriction sites 5' of the ribosome-binding site and the CAT-coding sequences but, by the design of the oligonucleotide linker, does not serve as a promoter of CAT expression and does prevent translation of the CAT gene if mRNA was spuriously produced from an upstream promoter by the presence of a terminator codon in-frame. This fragment, carrying the cloning sites in the non-expressing CAT gene, is substituted for the expressed CAT gene in pSHW9 to produce plasmid chimera pCV1 as illustrated in Fig. 7. As expected, E. coli transformants harboring pCV1 prove to be chloramphenicol-sensitive. The plasmid pCV1 may be ob-

tained by above-described techniques known to the art from E. coli culture 39431 in the ATCC.

(h) pCV3

Plasmid chimera pCV3, as illustrated in Fig. 8, is created to remove the NarI sites of PBR322 that exist in the plasmid chimera pCV1 in addition to the NarI site introduced by the synthetic oligonucleotide linker. The other NarI sites were removed in pCV3 by eliminating the DNA between the BalI site of pBR322 and the PvuII site originating from pC194 DNA. Plasmid pCV3 may be obtained by above-described techniques known to the art from E. coli culture 39430 in the ATCC.

It is to be noted that plasmid pCV3 is of general applicability for the isolation of promoter-containing sequences in E. coli and in any other chloramphenicol-sensitive microorganism into which the nonexpressing CAT gene may be introduced, such as by means of a chimeric plasmid comprising pCV3 and another plasmid capable of replication in this microorganism.

(i) pBST110

Plasmid chimera pBST110, as illustrated in Fig. 9, is a plasmid chimera consisting of pCV3 and pBST2-6 which joins the two plasmids at their HindIII sites. The plasmid chimera pBST110 transforms both E. coli and Bacillus stearothermophilus, but does not confer chloramphenicol resistance to either.

The synthetic oligonucleotide in pBST110 is designed to permit shotgun cloning of Bacillus stearothermophilus DNA into the NarI site and selection for expression of the CAT gene driven by a promoter in the cloned DNA fragments. The cloned DNA fragments could be derived by NarI, ClaI, TaqI or MspI digestion of Bacillus stearothermophilus chromosomal or plasmid DNA, due to the same 5' overlapping bases left by all these enzymes. In addition, the cloned promoter could be excised as an EcoRI-BclI fragment, if desired, since these sites flank the NarI insertion site, and could therefore be re-cloned in a defined orientation to drive expression of another mesophilic, cryophilic or synthetic gene in Bacillus stearothermophilus. Alternatively, Bacillus stearthermophilus DNA could be inserted into either the EcoRI or BclI sites.

Plasmid chimera pBST110 is useful in the method for isolation of a promoter capable of functioning at elevated temperatures described above and may be obtained by above-described techniques known to the art from E. coli culture 39432 in the ATCC.

(j)  pRMS10

Plasmid chimera pRMS10 is derived from the shotgun-cloning of fragments obtained by digestion of B. stearothermophilus chromosomal DNA with the restriction enzyme Sau3AI inserted into the BclI site of pBST110 and selecting for the appearance of resistance to the antibiotic chloramphenicol. This phenotype is caused by the product of the CAT gene being able to degrade the antibiotic, and is manifested because a promoter is contained in the DNA fragment inserted such that the CAT gene is expressed. The sequence of the DNA insert containing the promoter may be determined by analysis of the plasmid pRMS10 by the method of Maxam and Gilbert, infra. Plasmid pRMS10 may be obtained by above-described techniques known to the art from B. stearothermophilus culture 39428 in the ATCC.

Plasmid chimera pRMS10 is useful for the transforma-tion of E. coli and B. stearothermophilus to produce the transformants capable of producing the enzyme chloramphenicol acetyl transferase.

Transformation of Thermophilic Microorganisms

The present invention employs a method of transforming thermophilic microorganisms comprising:

(a) forming a protoplast from a thermophilic microorganism;

(b) introducing a plasmid chimera containing genetic material from a source other than a thermophilic organism into the protoplast; and

(c) causing regeneration of the cell wall of the protoplast.

The transformation process of the present invention may be used to effect transformation and cell wall regeneration under conditions allowing identification of successfully transformed organisms in an abbreviated period of time, e.g., about two days following transformation.

The present invention further includes a method of producing a stable biological product at elevated temperatures corresponding to the product of non-thermophilic genetic material comprising:

(a) transforming a thermophilic microorganism by the method comprising:

(1) forming a protoplast from a thermophilic microorganism;

(2) introducing a plasmid chimera into the protoplast which contains genetic material from a source other than a thermophilic organism;

(3) causing regeneration of the cell wall of the protoplast; and

(b) placing the transformed thermophilic microorganism on a growth medium sufficient to cause the microorganism to multiply and to cause expression at elevated temperatures of a stable biological product corresponding
to the introduced genetic material.

The method of producing a thermostable enzyme of the
present invention is distinguished from hitherto known
methods in that, inter alia, the method provides transformed thermophilic bacteria which express the thermostable
enzyme within a shortened period of time, e.g. about two
days, of being placed on the growth medium.

The method of the present invention contemplates the
use of any thermophilic microorganism in the transformation
process.  Preferred thermophilic microorganisms for use
in the present invention include thermophilic bacteria
of the genus Bacillus.  Particularly, preferred thermophilic bacteria for use in the present invention are those
of the species Bacillus stearothermophilus.

To practice the method of the present invention to
transform thermophilic microorganisms, protoplasts are
generated by growing the microorganisms in medium capable
of physiologically supporting the microorganisms and allowing them to multiply.  Preferably, TSY medium, containing
tryptone, yeast extract and sodium chloride may be used
to grow the microorganisms.

The microorganisms are preferably grown at a temperature sufficient to support growth of only thermophilic
organisms.  Such a growth temperature is generally above
about 50°C.  When Bacillus stearothermophilus is the thermophilic microorganism to be transformed, the growth temperature is preferably about 55°C.

The microorganisms are grown until a workable concentration is obtained.  The optional concentration to which
the microorganisms are grown will vary depending on the
growth characteristics of the thermophilic bacterium sele-

0138075

cted and can be readily ascertained by those of ordinary skill in the art in light of the teachings contained herein and the inferences logically to be drawn from them. It is preferred that the microorganisms be in a concentration of about $0.5 \times 10^8$ to about $2.0 \times 10^8$ cells/ml, and most preferably in a concentration of about $1.0 \times 10^8$ cells/ml.

After the microorganisms have been grown to the optimal concentration, the microorganisms are harvested from the growth medium. The harvesting is generally accomplished by centrifugation. However, other methods of separating cells from medium may be used. Other harvesting methods include, but are not limited to, filtration or, if the microorganisms have been grown on a solid agar support surface, scraping of the cells off the agar surface. In the preferred method of the present invention, the cells are harvested by centrifugation. The harvesting is to be accomplished at a temperature selected to ensure continued viability of the organisms, but it is not necessary to further the growth or multiplication of the microorganisms at this stage. Preferably, the microorganisms are harvested at about room temperature.

After harvesting, the microorganisms are resuspended in medium which will physiologically support the microorganisms and which contains an osmotic stabilizer to ensure that the integrity of the cell membranes of the protoplasts, when created, will be maintained. For example, medium containing the necessary components for growth, in addition to an osmotic stabilizer, would be suitable. Preferably, the microorganisms of the present invention are resuspended in SMMX medium, a medium composed of tryptone, yeast extract, sodium chloride, glucose, sucrose, sodium maleate, magnesium chloride and bovine serum albumin. The amount of medium used to resuspend the microorganisms will vary depending on the number of microorganisms which are to be resuspended, however, in the preferred embodiment the microorganisms are resuspended in one-tenth of the volume used to cause growth.

The resuspended microorganisms are converted to protoplasts by the addition of a chemical substance which is capable of degrading the cell wall of the microorganism while allowing the microorganism to remain viable in the presence of an osmotic stabilizer. In the preferred embodiment, it is contemplated that lysozyme will be used as the chemical substance added to convert the microorganisms to protoplasts, although other substances such as protease, glycine or penicillin may be equally useful. It is preferred, when lysozyme is the added chemical degrading substance, to use highly purified egg-white lysozyme.

The concentration of the chemical degrading substance to be added varies with the concentration of the microorganisms and the chemical purity of the degrading agent. In the preferred embodiment of the present invention, lysozyme may be added to achieve a lysozyme concentration of about 10 to about 500 $\mu$g/ml although concentrations of lysozyme of from 1 to 500 $\mu$g/ml may be useful. Most preferably, lysozyme is added to achieve a lysozyme con- centration of about 50 $\mu$g/ml.

After the addition of the chemical degrading substance, the microorganisms are incubated for a period of time sufficient to cause the cell wall of the microorganism to be degraded by the chemical substance. The time period of the incubation will be one that is necessary for the cell walls to be degraded but not so long as to cause the viability or cell membrane of the microorganisms to be impaired. Preferably, the microorganisms will be incubated in the presence of lysozyme for about 5 to about 30 minutes, preferably about 15 minutes.

The incubation is conducted at a temperature sufficient to sustain the viability of the protoplasts. Such an incubation temperature may range from about 4°C to about 50°C, preferably about 4°C to about 47°C. Most preferably, the incubation temperature is about 37°C.

At the end of the incubation period, the protoplasts are separated from the chemical degrading substance. This separation is accomplished by any separation method discussed above, preferably by centrifugation. After the chemical degrading substance has been removed, the protoplasts are resuspended in medium containing an osmotic stabilizer. Preferably, SMMX medium is used to resuspend the protoplasts.

The method of the present invention for creating protoplasts provides for a conversion of cells to protoplasts that is virtually complete. In the method of the present invention, fewer than one cell in $10^5$ remained viable following dilution and growth in the absence of an osmotic stabilizer.

The plasmid chimeras are introduced into the protoplasts by a transformation process. The transformation process of the present invention involves the mixing of the plasmid chimeras in a buffer selected to maintain the osmotic stability of the protoplasts and to supply an environment with a pH sufficient to support microbial viability. The preferred buffer for use in the present invention is a TE buffer, comprising 10 mm Tris-HCl; 1 mm EDTA, pH 8.0; and an equal volume of 2X SMM solution.

The protoplast suspension is added to the plasmid chimera and buffer mixture. Immediately after the addition of these two products, a transforming solution is added. Preferably, the transforming solution contains polyethylene glycol (PEG) as an active ingredient. Any PEG having a molecular weight sufficient to cause transformation may be used.  Preferably 8000 molecular weight PEG is used in a concentration of 40% weight/volume (w/v) in SMM buffer.

The PEG solution is prewarmed to a temperature ranging from 40 to 60°C, preferably 47°C.  The addition of the prewarmed PEG constitutes the beginning of a "heat-pulse" period.  The heat-pulse is continued for 0.5 to 15 minutes, preferably for 2 minutes.

At the end of the heat-pulse period, the protoplasts are separated from the transforming solution.  This separation is contemplated as being a step which does not occur instantaneously.  In the preferred embodiment, the separation step is accomplished by centrifugation.  The preferred centrifugation may be accomplished during a time span ranging in length from 5 to 15 minutes.  It is preferred that the centrifugation be accomplished over a time span of eight minutes.  The centrifugation is accomplished at a temperature lower than that of the heat-pulse step but high enough so as to maintain protoplast viability.  Preferably, the centrifugation is accomplished at a temperature between 4 and 47°C.  Most preferably, the centrifugation is accomplished at room temperature.

At the end of the incubation period and centrifugation, medium is added to resuspend the protoplasts in the reaction vessel.  It is contemplated in the present invention that the medium added to dilute the protoplast solution be medium designed to maintain protoplast viability and osmotic stability, preferably SMMX medium.

Once the protoplasts have been resuspended, it is desirable to allow a resting period for regeneration of the cell wall and expression of the transformed phenotype to occur.  In the present invention, it was found that a period ranging from about 1 to 1.5 hours is preferable. It was also found to be desirable to conduct the resting period at an elevated temperature. Preferably, temperatures ranging from about 45 to 60°C are used. Most preferably, an incubation temperature of about 47°C is used. In addition, it is preferable to have the vessel gently shaken during the regeneration period.

In the preferred embodiment of the present invention, novel medium, DM3a, is used as a regeneration medium. Table II sets forth the composition of the novel DM3a medium. The DM3a medium provides, in the preferred embodiment of the present invention, in addition to the ingredients set forth in Table II, an appropriate antibiotic or antibiotics to allow detection of the transformed phenotype now present in the regenerated microorganisms due to the plasmid chimera that was introduced.

Table II

Composition of Protoplast Regeneration Media DM3a

| | Concentration |
|---|---|
| Na Succinate (pH 7.3) | 0.15M |
| Agar | 2% |
| Casamino acids | 0.5% |
| Yeast Extract | 0.5% |
| $K_2HPO_4$ | 0.35% |
| $KH_2PO_4$ | 0.15% |
| Glucose | 0.5% |
| MgCl2 | 0.02M |
| Bovine Serum Albumin | 0.01% |
| Kanamycin (when required) | 25 $\mu$g/ml |
| Chloramphenicol (when required) | 1 $\mu$g/ml |

At the end of the resting period, the microorganisms are allowed to regenerate cell walls and multiply in an environment appropriate for the physical manifestations of the acquired genetic material to become apparent. The microorganisms are preferably plated onto solidified regeneration medium containing essential growth nutrients and any necessary substance to enable one to visualize which microorganisms were successfully transformed. It is preferred that the regeneration medium contain as many enrichment factors as possible. It is also preferred that the regeneration medium contain sodium succinate as an osmotic stabilizer.

When, as in the preferred embodiment, the microorganisms are plated onto DM3a medium with an antibiotic corresponding to the trait conferred during the transformation, successful transformants will exhibit growth in the presence of the antibiotic. The decreased times necessary to score transformants observed when using the method of the present invention is believed made possible by the enriched regeneration medium DM3a.

When using the method of the preferred embodiment, transformants will be obvious within two days from the time the transformants are plated on the DM3a medium. Approximately, 10% of the protoplasts can be regenerated using the present invention as set forth above.

Method of Creating Thermostable Variants of Biological Products.

The process of the present invention sets forth a method to be used for creating thermostable variants of mesophilic, cryophilic, thermophilic or synthetic biological products. This process comprises: (a) the insertion into a cloning vector of a DNA sequence corresponding to the biological product; (b) causing the sequence coding for the biological product to be mutagenized; (c) transforming a microorganism capable of growth at an elevated temperature with the cloning vector; (d) growing the transformed microorganism at elevated temperatures to cause expression of the biological product; and (e) identifying the organisms growing at elevated temperatures and expressing the variant form of the biological product.

The cloning vector to be used may be any that is suitable for use in an organism which grows at an elevated temperature. It is preferred that the cloning vector used be able to direct expression of the genetic material coding for the biological product in more than one genus of microorganisms. It is most preferred that the cloning vector be one which allows for expression of the genetic material coding for the enzyme in both a mesophile, for example, E. coli, and a thermopile, for example, Bacillus stearothermophilus.

An example of a preferred cloning vector in Bacillus stearothermophilus and E. coli is the plasmid chimera pRMS10 described above. These plasmid chimeras are preferred because they contain a promoter capable of directing expression of the genetic material at an elevated temperature, a ribosome-binding site, an origin of replication which is functional at an elevated temperature, and convenient cloning sites in which to insert a gene of interest. The most preferred cloning vector is pRMS10.

The gene to be inserted in the cloning vector is any which one wishes to have expressed and active at an elevated temperature. In one embodiment of the present invention, genes which code for biological products of industrial significance will be inserted.

The genes to be inserted into the cloning vector may be obtained in any feasible manner. In one embodiment of the present invention, the genes may be obtained from a microorganism. In another embodiment of the present invention, the genes to be inserted into the cloning vector may be synthesized. If synthesized, any acceptable synthesis method may be used. An example of an acceptable method is that developed by Caruthers, M. H., Beaucage, S. L., Becker, C., Efcavitch, W., Fisher, E. F., Gallupi, G., Goldman, R., deHaseth, P., Martin, F., Matteucci, M., and Stabinsky, Y., in Genetic Engineering, pp 1-17, eds. Setlow, J. K., and Hollaender, A., Plenum Press, New York, 1982, the disclosure of which is specifically incorporated herein by reference. A benefit of the utilization of synthesized genes is that the genetic material which is inserted will be limited to the actual amino acid-coding sequences. In this embodiment, gene expression would be derived from promoters and ribosome binding sites chosen for this purpose.

Mutagenesis may be induced in a variety of manners. Mutagenesis may be in vivo or in vitro. In vivo mutagenesis may occur by chemical inducement, radiation, or ultraviolet means, or by passage of the plasmid through a cell with a high frequency of causing mutations.

In vitro mutagenesis, as contemplated by the present invention, may be induced by any suitable technique, including, but not limited to, chemical inducement or radiation inducement. Preferably, when the in vitro mutagenesis is induced, the DNA sequences comprising the original cloning vector, not including the inserted genetic material, is protected by maintaining double-strandedness Such double-strandedness may be maintained by forming heteroduplex molecules between single strands of vector alone and of vector with cloned gene. Such a heteroduplex molecule would allow mutagenesis to occur only in the area of single-strandedness; that is, in the area of the cloned gene. In addition, by using the preferred method of bisulfite treatment (K. W. C. Peden and D. Nathaus, Proc. Natl. Acad. Sci. USA 79: 7214-7217, December (1982)) to induce mutagenesis, a large number of molecules can be mutagenized at one time. Also, the extent of mutagenesis is readily controllable. The method contemplated by the present invention further avoids spurious effects in the subsequent selection for thermostable variants, such as increased gene copy number or increased rates of expression which would compensate for an intrinsic thermal lability of the enzyme.

Once the mutagenesis has been caused by any method known to those experienced in the art, the cloning vector is transformed into the microorganism using the above disclosed method for transformation of thermophilic microorganisms. The desired variants of the transformed thermophilic microorganisms are then identified by one of many methods. One possible identification method involves the growth, at an elevated temperature, of the transformants on medium which allows successful transformants to be visible. Preferably, this medium will allow only successful transformants to survive and multiply; successful transformants will contain thermostable variants of the mutagenized gene. In this method, single colonies possessing the desired activity will be identifiable and can be isolated.

A second identification method for selection of the desired variant is to grow the transformants while gradually changing the environmental conditions. This method is designed to force a selection of the desired variant. An example would be to grow the transformants in an environment which causes a continuous increase in temperature. In this method, only successful transformants would survive when the ultimate temperature was reached.

A third method for the identification of the desired variant is to screen for the enzymatic activity or for structural characteristics of the enzyme in the transformants grown at elevated temperatures, without prior selection for the activity per se. Such a screening procedure may, for example, be conducted by breakage of the cells and assaying for the appropriate enzymatic activity.

Alternatively, the screen may be done utilizing immunological techniques for the detection of products of cloned gene. An example of such an immunological technique is disclosed in L. Clarke, R. Hitzeman and J. Carbon, Methods in Enzymology, 68, 436-442 (1976) the disclosure of which is specifically incorporated herein by reference. In this method, the enzyme would be first purified from its original mesophilic, cryophilic or thermophilic source, and antibodies would be raised against the purified protein. Classes of antibodies that recognize structural domains of the protein rather than just simple stretches of amino acids, and which do not recognize the polypeptide corresponding to the denatured enzyme, would be used to probe transformant colonies grown at elevated temperatures for the presence of the structural antigenic determinants of the enzyme.

In a fourth method, thermostable variants of the enzyme of interest may be selected for indirectly. This would be accomplished by fusing the genes encoding for the enzyme with that encoding a function that may be selected for, in such a manner as to produce a fusion protein product which manifests the selectable activity only if the entire fusion product is thermostable. Such fusion proteins are analogous to those that may be obtained by, for example, the method of M. Rose and D. Botstein, Methods in Enzymology 101, 167-180 (1983) the disclosure of which is specifically incorporated herein by reference.

Different results may be obtained depending on the alternative used for selection of the desired variant; one method could yield an enzyme which has higher activity at higher temperatures, whereas another method could yield an enzyme which is more stable at higher temperatures.

While the foregoing embodiments have been described with specific reference to formation and identification of mutagenized genes having enhanced thermal stability, it will be obvious to those having ordinary skill in the art that the technique is generally applicable to formation and identification of enzymes tolerant to a wide variety of normally hostile conditions. Thus, with appropriate modification of the screening technique, genes coding for enzymes which are tolerant to acid conditions, salts, low temperatures, etc. may be prepared and identified.

The present invention may also be used to create biological products from a microorganism exhibiting a desired microbial phenotype comprising:

(a) the insertion into a cloning vector of a DNA sequence corresponding to said biological product;

(b) causing said sequence coding for said biological product to be mutagenized;

(c) transforming a microorganism with the cloning vector;

(d) growing said transformed microorganism in an environment capable of producing the desired microbial phenotype; and

(e) identifying the microorganisms which both express the desired phenotype and produce said biological product. This method is useful in creating biological products in, for example, halophilic, cryophilic or acidophilic microorganisms.

The following examples serve to elucidate the teachings of the present invention, and in no way limit the scope of the invention. Various other modifications and equivalents of the examples will readily suggest themselves to those of ordinary skill in the art, particularly after the issuance of this patent, without departing from the spirit or scope of the present invention.

Example 1 - Transformation of Thermophilic Microorganisms

Staphylococcus aureus plasmid pUB110 (kan$^R$) which transforms B. subtilis was used to demonstrate transformation in B. stearothermophilus.

To transform Bacillus stearothermophilus, protoplasts in SMMX medium (comprised of the components of SMM buffer, namely 0.5 M sucrose, 0.02 M Na maleate, 0.02 M $MgCl_2$, pH 6.5, and, in addition, 2% tryptone, 1% yeast extract, 1% NaCl, 0.5% glucose and 0.05% bovine serum albumin) were formed by the addition of lysozyme. Lysozyme, obtained from Miles Laboratories, was added at a concentration of 50 $\mu$g/ml. After the addition of the lysozyme, the microorganisms were incubated at 37°C for 15 minutes. After the end of the incubation period, the protoplasts were washed twice with SMMX medium by centrifugation at 1400 RPM for 8 minutes and resuspended in SMMX. Under these conditions, the conversion of cells to protoplasts was virtually complete (fewer than one cell in $10^5$ remained viable following dilution and plating in the absence of an osmotic stabilizer).

The plasmid chimeras were introduced into the protoplasts by the following transformation reaction: The plasmid chimeras were mixed with 50 $\mu$l of TE buffer (10 mm Tris-HCl, 1 mm EDTA, pH 8.0) and an equal volume of 2x SMM solution in a transformation reaction vessel. Then 0.5 ml of the protoplasts suspension was added to the vessel, followed by the immediate addition of 1.5 ml prewarmed (47°C) PEG solution (Sigma 8,000 average molecular weight, 40% w/v in SMM buffer). The transformation reaction vessel was incubated at 47°C for 2 minutes after the addition of the PEG, at which time 5 ml SMMX was added to dilute the solution and the protoplasts were centrifuged out of solution at room temperature. The pelleted, transformed protoplasts were resuspended with 0.5 ml SMMX and incubated at 47°C for 1 to 1.5 hours, with gentle shaking, to allow expression of the transformed phenotype.

The transformants were ultimately plated on DM3a medium containing the 25 μg/ml kanamycin at 47°C to allow regeneration of the cell walls and selection for the presence of pUB110. The composition of DM3a medium is set forth in Table II. Rapid screening of transformants of B. stearothermophilus for presence of plasmids was done using an alkaline extraction procedure using the method of H. C. Birnbaum and J. Doly, Nucleic Acids Research 7: 1513-1523 (9179), the disclosure of which is specifically incorporated herein by reference.

Plasmid DNA was prepared from B. stearothermophilus as follows: 2L cultures in TSY containing the appropriate selective drug were grown up overnight at 47°C, harvested by centrifugation, washed with 250 ml 10 mM Tris-HCl, pH 8.0/0.1 M NaCl/1 mM EDTA, and resuspended with 44 ml 50 mM Tris-HCl, pH 8.0/50 mM EDTA containing 1 mg/ml lysozyme. The cell suspension was incubated at 37°C for 30 min. and lysed by the addition of 1 ml 10% SDS. Following the addition of 10 ml 5M NaCl, the lysate was kept on ice for at least 1 hour before centrifugation in a Beckman SW27 (25,000 rpm, 45 min) or Ti60 (30,000 rpm, 45 min rotor). The plasmid DNA was twice-banded by CsCl-ethidium bromide gradient centrifugation and recovered as described in "Advanced Bacterial Genetics", by R. W. Davis, D. Botstein and J. R. Roth, Cold Spring Harbor Laboratory, Cold Spring Harbor, New York, the disclosure of which is specifically incorporated herein by reference.

When analyzed by restriction endonuclease digestion, pUB110 re-isolated from B. stearothermophilus transformants were essentially identical to the plasmids originally purified from B. subtilis. A notable exception was that certain, but not all, MboI sites in the B. stearothermophilus-derived DNA's were modified such that these sites were no longer susceptible to cleavage by this enzyme and were cut only by Sau3AI.

Example 2 - Construction of pBST2

Five $\mu$g of pUB110 DNA was digested with 10 units of the restriction endonuclease TaqI in 30 $\mu$l of 10 mM Tris-HCl, pH 7.4/10 mM MgCl$_2$/50 mM NaCl at 65°C for 1 hr. The reaction mixture was extracted with phenol followed by ether. The DNA was precipitated with ethanol and resuspended with 50 $\mu$l 10 mM Tris-HCl, pH 7.4/1 mM EDTA. Similarly, 1 $\mu$g of pBST1 DNA was digested with ClaI in 30 $\mu$l 10 mM Tris-HCl, pH7.4/10 mM MgCl$_2$ at 37°C for 1 hr. and extracted and precipitated as above. The digested pBST1 DNA was resuspended with 20 $\mu$l 10 mM Tris-HCl, pH7.4/1 mM EDTA. Fifteen $\mu$l of the digested pUB110 DNA were mixed with 7 $\mu$l of the digested pBST1 DNA and ligation was promoted by the addition of 800 units of T4 DNA ligase in a reaction mix of final volume of 50 $\mu$l composed of 50 mM Tris-HCl, pH 7.4/1 mM MgCl$_2$/1 mM adenosine triphosphate/ 10 mM dithiothreitol. The ligation reaction was carried out at 4°C for 16 hr., and 40 $\mu$l were subsequently used to transform B. stearothermophilus as described in example 1, selecting for colonies resistant to 25 $\mu$g/ml kanamycin. Four such colonies were obtained and were passaged on drug-free plates at 70°C, following which they were again tested for kanamycin resistance at 47°C. One of these colonies was found to be still resistant to the antibiotic, and shown to possess a plasmid containing DNA segments from both parental pUB110 and pBST1 plasmids as described above.

Example 3 - Construction of pBST2-6

Three $\mu$g of pBST2 DNA were digested with 100 units EcoRI in a 50-$\mu$l reaction mixture composed of 50 mM Tris-HCl, ph 7.4/10 mM MgCl$_2$/100 mM NaCl at 37°C for 1 hour, and treated subsequently with 4 units of calf intestinal alkaline phosphatase at 30°C for 15 min. The DNA was then phenol extracted, ethanol precipitated, and resuspended with 20 μl 10 mM Tris-HCl/1 mM EDTA as described in the previous construction. Synthetic DNA chains were prepared by the phosphite triester method disclosed in Caruthers, M. H., Beaucage, S. L., Becker, C., Efcavitch, W., Fisher, E. F., Gallupi, G., Goldman, R., deHaseth, P., Martin, F., Matteucci, M., and Stabinsky, Y., in Genetic Engineering, pp 1-17, eds. Setlow, J. K., and Hollaender, A., Plenum Press, New York, 1982. The synthetic linker was prepared for ligation to the EcoRI-digested pBST2 as follows: 1 nanomile of each of the synthetic DNA chains comprised of the sequences 5'-AATTCAAGCTT-3' and 5'-AATTAAGCTTG-3' were mixed in 10 μl 10 mM Tris-HCl/1 mM EDTA, heated at 70°C for 10 min, and cooled to 4°C over a period of 3 hours to promote duplex formation.

Five μl of the annealed linker were then taken with 4 μl of the cut plasmid and ligation with T4 DNA ligase. Unreacted linkers and multimers thereof were removed from the reaction mix by HPLC chromatography on TSK-G5000PW medium (see M. E. Himmel, P. J. Perna and M. W. McDonnell, Journal of Chromatography, 240: 155-163 (1982), which is specifically incorporated by reference herein). The purified DNA was then cleaved with EcoRI to remove any multimers incorporated into the plasmid. This DNA was rejoined with phage T4 DNA ligase and transformation of B. stearothermophilus performed as described in example 1.

Example 4 - Construction of pSHW9

The hybrid plasmid pSHW9 was constructed by digesting pC194 with MspI and subsequently also with Sau3AI, and ligating its fragments to those generated by digesting pBR322 with ClaI followed by BamHl, taking advantage of the fact that the restriction enzymes MspI and ClaI leave complementary overhangs, as do the enzymes Sau3AI and BamHl. The procedure for the digestions and subsequent ligation reaction were accomplished as in examples 2 and 3.

The products of the ligation reaction were transformed into E. coli according to the method of Davis, et al. Ibid., the disclosure of which is specifically incorporated herein by reference, and transformants selected on 5 μg/ml chloramphenicol. A candidate transformant was shown to harbor a hybrid plasmid composed of DNA segments from pC194 and pBR322.

Example 5 - Construction of pBST8

The hybrid shuttle vector plasmid pBST8 was constructed by ligating EcoRI digestion products of pBST2-6 and pSHW9 using procedures described in Examples 2-4. Portions of the ligation mixtures were transformed into E. coli as well as B. stearothermophilus and identical products obtained. Since the plasmid in E. coli exists in higher copy number than in B. stearothermophilus, it was found to be more convenient to prepare purified plasmid from the former organism for analyses and further constructions.

Example 6 - Isolation of Thermophilic Promoter

The chimeric plasmid pBST110, composed of the chimeric plasmids pCV3, which is designed to allow the identification of cloned segments of DNA that act as promoters, and of the chimeric plasmid pBST2-6, which incorporates a thermostable origin of replication, was digested with the restriction enzyme BclI. Into this site was shotgun-cloned fragments of B. stearothermophilus chromosomal DNA obtained by Sau3AI digestion. The clones were transformed into E. coli and plasmids prepared from all the transformants collectively. This heterogeneous mixture of plasmids containing B. stearothermophilus DNA constitutes a gene bank from this organism. One microgram of this mix of plasmids was used to transform B. stearothermophilus and a random 672 kan[R] transformants tested for the cam[R] phenotype that would arise from the presence of a promoter in the cloned DNA sequences. Thirteen such transformants were resistant to chloramphenicol and their plasmids were analyzed. A representative plasmid, pRMS10, was isolated from the B. stearothermophilus transformant and transformed into E. coli. A large amount of plasmid was purified from this organism and the insert DNA may be sequenced according to the method of Maxam and Gilbert, Methods in Enzymology, vol. 65, 499-560, (1980) the disclosure of which is specifically incorporated herein by reference.

Example 7 - Thermophilic Drill

The enzyme kanamycin nucleotidyl transferase, which confers resistance to the antibiotic kanamycin, was used to demonstrate the operability of the process whereby enzyme variants which are active and stable at elevated temperatures may be produced from enzymes which are not normally active or stable at these temperatures. The gene encoding this enzyme is present on pBST2-6, being derived from pUB110 DNA sequences. Thus, a B. stearothermophilus transformant harboring pBST2-6 was plated on solid TSY medium containing 20 $\mu$g/ml kanamycin at $10^8$ cells per plate, and incubated at 66°C. This transformant normally is not resistant to the antibiotic at this temperature, but is only resistant at up to 55°C. A colony was isolated under these conditions and was shown to be able to grow reproducibly in the presence of kanamycin at elevated temperatures. The plasmid purified from this strain was able to transform B. stearothermophilus to kanamycin resistance at the high temperature, indicating that the high-temperature kanamycin resistance was plasmid-encoded and not due to modification of the genetic cor-sition of the cells harboring the plasmid. This plasmi:, being a variant of pBST2-6, is denoted pBST2-6TK. Assay of the enzymatic kanamycin nucleotidyl transferase activity in normal and in the high-temperature kanamycin-resistant cells indicated that the enzyme in the latter cells was significantly more thermostable: the activity decayed with a halflife of 42 minutes when incubated at 55°C whereas under identical conditions the wildtype activity decayed with a halflife of 4.5 minutes.

The thermophilic variant with enhanced stability was obtained without mutagenesis, that is, was the result of a spontaneous mutational event; spontaneous mutations do occur at low frequency. However, the intervention of mutagenesis will greatly increase the frequency at which such variants are produced.

It will be apparent to those skilled in the art that various modifications and variations can be made in the processes and products of the present invention. Thus, it is intended that the present invention cover the modification and variations of this invention provided they come within the scope of the appended claims and their equivalents.

WHAT IS CLAIMED IS:

1. A method for transformation of thermophilic microorganisms comprising the steps of:

(a) forming a protoplast from a thermophilic microorganism;

(b) introducing a plasmid chimera into said protoplast which contains genetic material from a source other than a thermophilic organism; and

(c) causing regeneration of the cell wall of said protoplast.

2. A method of producing a thermostable enzyme comprising:

(a) transforming a thermophilic microorganism by the method comprising:

(1) forming a protoplast from a thermophilic microorganism;

(2) introducing a plasmid chimera into the protoplast which contains genetic material from a source other than a thermophilic organism;

(3) causing regeneration of the cell wall of the protoplast; and

(b) placing the transformed thermophilic microorganism on a growth medium sufficient to cause the microorganism to multiply and to cause expression, at elevated temperatures, of a thermostable biological product.

3. The method of claim 1 wherein said plasmid is introduced into said protoplast in a polyethylene glycol solution.

4. The method of claim 3 wherein said polyethylene glycol solution is prewarmed to 47°C.

5. The method of claim 1 wherein said plasmid contains synthetic genetic material.

6. The method of claim 1 wherein said plasmid contains a thermostable origin of replication.

7. The method of claim 1 wherein said plasmid contains a promoter that functions at elevated temperatures.

8. The method of claim 1 wherein said plasmid contains a synthetic oligonucleotide linker.

9. The method of claim 1 wherein said regeneration of the cell wall of said protoplast occurs in medium with 0.15 M sodium succinate.

10. A method of claim 1 wherein said medium used for regeneration of said cell walls of said protoplasts is DM3a medium.

11. The method of claim 1 wherein said thermophilic microorganism to be transformed is a bacterium.

12. The method of claim 1 wherein said thermophilic microorganism to be transformed is a gram-positive bacterium.

13. The method of claim 1 wherein said thermophilic microorganism to be transformed is of the genus Bacillus.

14. The method of claim 1 wherein said thermophilic microorganism to be transformed is Bacillus stearothermophilus.

15. A thermostable plasmid capable of replication and phenotypical expression of genetic material obtained from a non-thermophilic organism.

16. The thermostable plasmid of claim 15 wherein said plasmid comprises a thermostable origin of replication.

17. The thermostable plasmid of claim 15 wherein said plasmid comprises a thermostable promoter.

18. The thermostable plasmid of claim 15 wherein said plasmid comprises genetic material obtained from a non-thermophilic microorganism.

19. The thermostable plasmid of claim 15 wherein said genetic material is obtained from a microorganism of the genus Bacillus.

20. The thermostable plasmid of claim 15 wherein said genetic material is obtained from a microorganism of the genus Escherichia.

21. The thermostable plasmid of claim 15 wherein said plasmid comprises genetic material obtained from a cryophilic organism.

22. The thermostable plasmid of claim 15 wherein said plasmid comprises synthetic genetic material.

23. The thermostable plasmid of claim 15 wherein said plasmid comprises a synthetic oligonucleotide linker.

24. The thermostable plasmid of claim 23 wherein said synthetic oligonucleotide linker has the DNA sequence shown in Figure 2.

25. The plasmid pBST1.

26. The plasmid chimera pBST2.

27. The plasmid chimera pBST2-6.

28. The plasmid chimera pBST2-6TK.

29. The plasmid chimera pSHW9.

30. The plasmid chimera pBST8.

31. The plasmid chimera pCV1.

32. The plasmid chimera pCV3.

33. The plasmid chimera pBST110.

34. The plasmid chimera pRMS10.

35. A promoter sequence comprising a DNA sequence capable of insertion into a plasmid vector containing non-thermophilic genetic material, which vector is capable of transforming thermophilic microorganisms, said DNA sequence being capable of initiating expression of said non-thermophilic genetic material in said thermophilic microorganisms at elevated temperatures.

36. A promoter sequence according to claim 35 wherein said promoter comprises the DNA sequence acting as the promoter governing expression of the CAT gene in plasmid pRMS10.

37. A thermostable origin of replication comprising DNA obtained by ClaI digestion of pBST1.

38. A method for isolation of a promoter that functions at elevated temperatures, comprising:

(a) construction of a plasmid chimera useful for determining whether an inserted piece of DNA is a promoter that functions at elevated temperatures, said plasmid chimera comprising:

(1) a genetic sequence capable of expressing a detectable phenotype;

(2) a ribosome-binding site capable of operation at elevated temperatures; and

(3) an insertion site wherein a piece of DNA may be inserted such that said piece of DNA would, if capable, act as a promoter for said genetic sequence;

(b) inserting said piece of DNA suspected of being a promoter that functions at elevated temperatures into said insertion site;

(c) transforming said plasmid chimera into thermophilic microorganisms; and

(d) identifying said microorganisms which express the phenotype corresponding to said genetic sequence.

39. The method of claim 38 wherein said plasmid chimera is pCV1.

40. The method of claim 38 wherein said plasmid chimera is pCV3.

41. The method of claim 38 wherein said plasmid chimera is pBST110.

42. The method of claim 38 wherein said plasmid chimera is pRMS10.

43. The method of claim 38 wherein said pieces of DNA are obtained from Bacillus stearothermophilus.

44. The method of claim 38 wherein said pieces of DNA are obtained through a synthetic synthesis.

45. A method for creating thermostable variants of biological products, comprising:

(a) inserting into a cloning vector a DNA sequence coding for said biological product;

(b) causing said sequence coding for said biological product to be mutagenized;

(c) transforming microorganisms capable of growth at an elevated temperature with said cloning vector;

(d) growing said transformed microorganism at elevated temperatures to cause expression of the mutagenized DNA sequence originally coding for said biological product; and

(e) identifying the microorganisms which both grow at elevated temperatures and express thermostable variants of said biological product.

46. The method of claim 45 wherein said DNA sequence is obtained from a mesophilic microorganism.

47. The method of claim 45 wherein said DNA sequence is obtained from a cryophilic microorganism.

48. The method of claim 45 wherein said DNA sequence is synthetic.

49. The method of claim 45 wherein said cloning vector is pRMS10.

50. The method of claim 45 wherein said mutagenizing is caused by bisulfite.

51. The method of claim 45 wherein said biological product being expressed is kanamycin nucleotidyl transferase.

52. The method of claim 45 wherein said cloning vector is capable of expressing said biological product when transformed into a thermophile and a mesophile.

53. The method of claim 45 wherein said cloning vector is capable of expressing said biological product when transformed into a thermophile and a cryophile.

54. The method of claim 45 wherein said expression of said biological product occurs when said transformed microorganism is grown at an elevated temperature.

55. The method of claim 45 wherein said growing of said transformed microorganism at said elevated temperature occurs when said microorganism is put into an environment sustained at said elevated temperature.

56. The method of claim 45 wherein said growing of said transformed microorganism at said elevated temperature occurs when said microorganism is put into an environment where the temperature is gradually raised to said elevated temperature.

57. A method for creating variants of biological products having desired characteristics comprising:

(a) inserting into a cloning vector a DNA sequence coding for said biological product;

(b) causing said sequence coding for said biological product to be mutagenized;

(c) transforming a microorganism with said cloning vector;

(d) growing said transformed microorganism in an environment capable of inducing exhibition of the desired characteristics; and

(e) identifying the microorganisms which both exhibit the desired characteristics and produce said variants of said biological product.

58. The regeneration medium DM3a.

0138075

1/3

IN ALL FIGURES,

REPRESENTS DNA SEGMENTS FROM pBSTI

REPRESENTS DNA SEGMENTS FROM pBR322

REPRESENTS DNA SEGMENTS FROM pUBIIO

REPRESENTS DNA SEGMENTS FROM pCI94

*FIG. I.*

*FIG. 2.*

EcoRI   Hind III

AA TT C AA GC TT

G TT CG AA TT AA

*FIG. 3.*

NruI   EcoRI   XbaI   Bam HI   PvuI

SacII

pBST2
5·9 kb

Kan R   BglI

NcoI

FIG. 4.

2/3

0138075

pBST 2-6
5·9 kb

FIG. 5.

pSHW9
6·0

FIG. 6.

pBST8
11·9kb

0138075

## FIG. 7.

EcoRI     NarI     BclI

NcoI

AMPR

pCVI

ClaI
HindIII
PvuII
BamHI

PvuII

BalI

## FIG. 8.

EcoRI     NarI     BclI

NcoI

AMPR

pCV3
4.5

ClaI
HindIII

PvuII

## FIG. 9.

EcoRI     NarI     BclI

NcoI

PstI

AMPR

ClaI
EcoRI
HindIII
NruI

SacII

PvuII

pBSTIIO
10.4

HindIII
EcoRI

XbaI
BamHI
PvuII

KanR

BglII

European Patent Office

EUROPEAN SEARCH REPORT

0138075

Application number

| DOCUMENTS CONSIDERED TO BE RELEVANT | | | EP 84111001.8 |
|---|---|---|---|
| Category | Citation of document with indication, where appropriate, of relevant passages | Relevant to claim | CLASSIFICATION OF THE APPLICATION (Int. Cl.4) |
| D,A | JOURNAL OF BACTERIOLOGY, vol. 149, no. 3, March 1982, (Washington D. C.)<br><br>T. IMANAKA et al. "Transformation of Bacillus stearothermophilus with Plasmid DNA and Characterization of Shuttle Vector Plasmids Between Bacillus stearothermophilus and Bacillus subtilis" pages 824-830<br><br>  * Page 824, abstract *<br><br>-- | 1-3, 13,14 | C 12 N 15/00<br>C 12 N  9/00<br>C 12 N  1/20<br>//C 12 R  1:07<br>C 12 R  1:185 |
| D,A | MOLECULAR & GENERAL GENETICS, vol. 168, no. 1, 1979 (Springer-Verlag; Berlin, New York)<br><br>S. CHANG et al. "High Frequency Transformation of Bacillus subtilis Protoplasts by Plasmid DNA" pages 111-115<br><br>  * Totality *<br><br>---- | 1-3,13 | **TECHNICAL FIELDS SEARCHED (Int. Cl.4)**<br><br>C 12 N |

The present search report has been drawn up for all claims

| Place of search | Date of completion of the search | Examiner |
|---|---|---|
| VIENNA | 17-12-1984 | WOLF |